# EUROPEAN PATENT SPECIFICATION

(11) **EP 3 643 362 B1**
(45) Date of publication and mention of the grant of the patent: **05.08.2026**
(21) Application number: 19204068.1
(22) Date of filing: 18.10.2019
(51) Int. Cl.: A61Q 5/06, A61K 8/04, A61K 8/49, A61K 8/81

(54) **SHINE ENHANCING SPRAY COMPOSITION**
GLANZERHÖHENDE SPRÜHZUSAMMENSETZUNG
COMPOSITION DE PULVÉRISATION AMÉLIORANT LA BRILLANCE

(30) Priority: 23.10.2018 EP 18202119
(43) Date of publication of application: 29.04.2020
(73) Proprietor: Kao Germany GmbH, 64297 Darmstadt (DE)
(72) Inventor: Schmid, Sabine, 64297 Darmstadt (DE); Cajan, Christine, 64297 Darmstadt (DE); Uellner, Yuqi, 64297 Darmstadt (DE)

(56) References cited:
- EP-A1- 3 329 904
- WO-A1-2018/018488
- US-B2- 10 010 489
- DATABASE GNPD [online] MINTEL; 18 July 2017 (2017-07-18), ANONYMOUS: "Freezing Spray", XP055580627, retrieved from www.gnpd.com Database accession no. 4955621
- DATABASE GNPD [online] MINTEL; 16 March 2017 (2017-03-16), ANONYMOUS: "Hold + Volume Strong Hairspray", XP055580631, retrieved from www.gnpd.com Database accession no. 4685441
- DATABASE GNPD [online] MINTEL; 27 May 2016 (2016-05-27), ANONYMOUS: "Shine Lipstick", XP055581119, retrieved from www.gnpd.com Database accession no. 4023499
- DATABASE GNPD [online] MINTEL; 21 September 2017 (2017-09-21), ANONYMOUS: "Brilliance Conditioner", XP055581122, retrieved from www.gnpd.com Database accession no. 5116029

## Description

The present invention relates to a substantially anhydrous shine enhancing spray composition for keratin fibers, especially for human hair, comprising, in a cosmetically acceptable medium, one or more hair setting film forming polymers and a pyrrolidone carboxylic acid ester.

Substantially anhydrous compositions applied by spraying onto keratin fibers and especially hair have been used for setting said fibers to a certain shape for many years. They are based on a non-aqueous solvent and comprising at least one hair setting /film forming polymer. In order to realize certain flexibility to the formed film on the keratin fiber surface, usually surfactants, polyols, oils especially silicone oils are added. However, addition of these substances influences performance of the composition and causes reducing of the setting effect. Generally, it is known that the sprays used for setting hair do take away some of the natural hair shine so that the hair appears less shiny, and has therefore dull appearance. There is a great need for products providing shine and hair setting to keep the hair in desired shape and hold in the shape for a considerable period of time.

The present invention starts from the above known problems with the performance of the sprays, and aims at providing a spray composition which does not affect hair setting negatively and additionally enhances hair shine.

Pyrrolidone carboxylic acid and its esters are known in hair conditioners and being used for improving several hair properties. EP 2090291 discloses aqueous hair conditioning compositions comprising PCA and/or its salts, one or more PCA ester and a hair conditioning compound selected from quaternary ammonium polymers and surfactants. The document does not address any substantially anhydrous compositions.

WO2018018488A1 discloses a hair styling composition comprising a fixing and/or film-forming polymer, a fatty alcohol, and rose hip oil. The fixing polymer comprises homo- and copolymers of vinyl pyrrolidone.

The inventors of the present invention have unexpectedly found out that a substantially anhydrous composition comprising one or more pyrrolidone carboxylic acid ester, wherein the pyrrolidone carboxylic acid ester is octyldodecyl pyrrolidone carboxylic acid, and one or more hair setting film forming polymers does provide good hair setting and additionally provides hair additional shine. It is usually the case that the compounds added into to the substantially anhydrous compositions take away part of hair setting effect and do result in less shiny hair. The inventors of the present invention have observed the opposite effects when the substantially anhydrous hair setting composition is added pyrrolidone carboxylic acid ester.

Accordingly, the first object of the present invention is a substantially anhydrous composition for keratin fibers, especially for human hair, comprising, in a cosmetically acceptable substantially anhydrous medium, one or more hair setting film forming polymers and one or more pyrrolidone carboxylic acid ester according to general structure 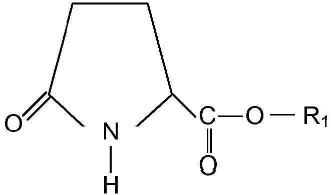
wherein R₁ is a straight or branched, saturated or unsaturated alkyl chain with 1 to 22
C atoms, phenyl, phenyl alkyl with alkyl chain length of 1 to 4 C atoms, phenyl alkoxy group with alkyl chain length of 1 to 4 C atoms and phenoxy alkyl group with alkyl chain length of 1 to 4 C atoms, wherein the pyrrolidone carboxylic acid ester is octyldodecyl pyrrolidone carboxylic acid.

The second object of the present invention is the use of the compositions for providing keratin fibers, especially human hair setting, hold and shine.

Then third object is the method of setting and shine enhancing keratin fibers, especially human hair wherein the dry hair is applied the composition of the present invention, preferably by spraying.

The fourth object is a kit for keratin fibers, especially human hair comprising the composition of the present invention.

Within the meaning of the present invention, the term "substantially anhydrous" means that the composition comprises no added water and the water concentration present in the composition may not exceed 5% by weight, preferably 3% by weight, more preferably 1% by weight and most preferably 0.5% by weight, calculated to the total composition. The water may be present in the compositions because the raw materials used comprised water which may be bound water. In particularly preferred form the compositions are anhydrous.

Within the meaning of the present invention, the term "hair setting film forming polymers" means that the polymer has setting effect on the hair by forming film on the hair surface.

The composition of the present invention comprises at least one pyrrolidone carboxylic acid ester of the above formula, wherein the pyrrolidone carboxylic acid ester is octyldodecyl pyrrolidone carboxylic acid which is available under the trade name Protelan ODPC from Zschimmer & Schwarz.

One or more pyrrolidone carboxylic acid esters is (are) comprised in the compositions of the present invention at a total concentration in the range of 0.01 to 15%, preferably 0.1 to 10%, more preferably 0.25 to 7.5% and most preferably 0.5 to 5% by weight, calculated to the total composition, in order to provide the desired effects.

The composition comprises one or more hair setting film forming polymers, preferably selected from anionic, cationic, non-ionic and amphoteric ones.

Non-limiting suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG.

Natural non-ionic polymers are as well suitable for the composition of the present invention. Those are such as starch, cellulose, chitosan, guar gum and their derivatives.

As amphoteric polymers which can be used alone or in mixture with at least one additional non-ionic polymer, reference is here made in particular to copolymers of N-octyl acrylamide, (meth)acrylic acid and tert.-butyl aminoethyl methacrylate of the type "Amphomer^{®}"; copolymers from methacryloyl ethyl betaine and alkyl methacrylates of the type "Yukaformer^{®}", e.g.. the butyl methacrylate copolymer "Yukaformer^{®} Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Non-limiting suitable anionic polymers alone or in combination with non-ionic polymers are vinyl alkyl ether, in particular methyl vinyl ether/maleic acid copolymers, obtained by hydrolysis of vinyl ether/maleic anhydride copolymers, distributed under the trade name "Gantrez^{®} AN or ES". These polymers may also be partly esterified, as for example, "Gantrez^{®} ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn^{®}"; sodium acrylate/vinyl alcohol copolymers of the type "Hydagen^{®} F", sodium polystyrene sulfonate, e.g.. "Flexan^{®} 130"; ethyl acrylate/acrylic acid/N-tert.-butyl acrylamide copolymers of the type "Ultrahold^{®}"; vinyl pyrrolidone/vinyl acetate/itaconic acid copolymers, acrylic acid/acrylamide copolymers or the sodium salts thereof of the type "Reten^{®}"; etc.

Further suitable anionic polymers are Acrylate copolymers available under trade name Salcare SC 81, PEG/PPG 25/25 dimethicone / acrylate copolymer available under trade name Luvigel Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Further suitable and preferred anionic polymer is acrylates/octylacrylamide copolymer available under the trade name Amphomer.

The anionic polymers are preferably neutralized either partially or completely, 100%, with an alkalizing agent. The suitable alkalizing agents are alkanolamines and especially preferred is aminomethlypropanol.

Non-limiting suitable cationic polymers are those known with their CTFA category name Polyquaternium. Typical examples of those are Polyquaternium-6, Polyquaternium-7, Polyquaternium-10, Polyquaternium-11, Polyquaternium-16, Polyquaternium-22 and Polyquaternium- 28, Polyquaternium-30, Polyquaternium-37, Polyquaternium-36, Polyquaternium-46, Polyquaternium-24, Polyquaternium-67, and Polyquaternium-72.

In this context, reference is also made to the cationic polymers disclosed in DE 25 21 960, 28 11 010, 30 44 738 and 32 17 059, as well as to the products described in EP-A 337 354 on pages 3 to 7, It is also possible to use mixtures of various cationic polymers.

The cationic polymers also include the quaternized products of graft polymers from organopolysiloxanes and polyethyl oxazolines described in EP-A 524 612 and EP-A 640 643. Among these especially preferred is the compound know with the INCI name Polysilicone-9.

The compositions comprise one or more hair setting film forming polymers at a total concentration in the range of 0.1 to 25% by weight, preferably 0.5 to 20% by weight, more preferably 1.0 to 15% by weight and most preferably 2 to 15% by weight, in particular 3 to 15% by weight, calculated to the total of the composition.

Substantially anhydrous composition of the present invention comprises organic solvents as the cosmetically acceptable medium. The compositions comprise one or more organic solvents. Suitable ones are ethanol, propanol, isopropanol, isopentane, n-pentane, n-hexane, dimethoxymethane, benzyl alcohol, benzyloxyethanol, alkylene carbonates such as ethylene carbonate and propylene carbonate, phenoxyethanol, butanol, isobutanol, cyclohexane, cyclohexanol, 1-phenylethylalcohol, 2-phenylethylalcohol, o-methoxyphenol. The preferred organic solvents are ethanol, isopropanol and propanol. The most preferred organic solvent is ethanol. The total concentration of one or more organic solvents is in the range of 30 to 95%, preferably 40 to 90%, more preferably 45 to 85%, most preferably 50 to 80% and in particular 60 to 75% by weight calculated to the total composition.

The composition of the present invention may be applied onto hair by spraying from a mechanical spraying device as well as form an aerosol device. In case an aerosol type of styling product is designed, the composition comprises additionally one or more propellants. Suitable propellants are lower alkanes such as n-butane, i-butane, propane, butane or their mixtures, as well as dimethylether (DME) either alone or in mixture with lower alkanes. Further suitable propellants are fluorinated hydrocarbons such as 1,1-difluoro ethane (Hydrofluorocarbon 152a) or tetrafluoroethane or their mixtures with each other, carbon dioxide and nitogen or their mixtures with the above mentioned propellants. Preferred are lower alkanes especially propane, butane, n-butane, i-butane, and their mixtures, 1,1-difluoro ethane (Hydrofluorocarbon 152a) and their mixture with DME at a alkane to DME weight ratio 10:1 to 1:10.

One or more propellants are comprised in the compositions at a concentration in the range of 10 to 80%, preferably 20 to 70%, more preferably 30 to 65% and most preferably 40 to 60% by weight, calculated to the total composition.

It should be noted all of the concentrations given in this specification for the components are calculated to the composition which may be non-aerosol composition without propellant or an aerosol composition with propellant.

In an embodiment of the present invention, compositions additionally comprise at least one polyol. The term polyol refers to a compound carrying 2 or more hydroxyl groups in its molecule.

Non-limiting suitable examples to the polyols preferred within the scope of the present invention are ethylene glycol and its homo polymers with varying molecular weight known with their INCI names such as diethyleneglycol, triethylene glycol and PEG with 4 to 800 ethylene glycol units, propylene glycol and its homo polymers with varying molecular weight known with their INCI names such as dipropylene glycol, tripropylene glycol and PPG with 4 to 50 propylene glycol units and glycerin and its homo polymers known with their INCI names diglycerin, triglycerin and polyglycerin with 4 to 40 gylcerin units. Any of the homopolymers mentione here can be liquid, semisolid, or solid. In the preferred from of the invention suitable polyols are the ones either liquid or semiliquid at ambient temperature. The most preferred are liquid ones at ambient temperature such as propylenegylcol, dipropylene glycol, PPG-9, diethylene glycol, triethylene glycol and glycerin.

The total concentration of one or more polyols in the composition is in the range of 0.01 to 2.5%, preferably 0.05 to 2%, more preferably 0.1 to 1.5% and most preferably 0.1 to 1% by weight, calculated to the total composition.

The composition may comprise one or more synthetic or natural oil (s). In principal, any oil allowed for cosmetic use is suitable for the compositions of the present invention.

Suitable oils are those of synthetic and of natural ones. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum are suitably contained within the scope of the present invention, It should as well be noted that compositions of the present invention can contain mixture of one or more natural oils and mineral oil.

Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions of the present invention. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401, DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

The total concentration of one or more oils is in the range of 0.01 to 1% by weight calculated to the total composition.

Additionally natural plant extracts can as well form part of the compositions of the present invention. Those are incorporated usually in an amount of 0.01 % to 0.5 %, by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon^{®}" products, "Herbasol^{R} ", "Sedaplant^{R}" and "Hexaplant^{R}". Extracts and the preparation thereof are also described in "Hagers Handbuch der pharmazeutischen Praxis". 4^{th} Ed..

Compositions of the present invention may comprise one or more UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysiliocne-15.

The suitable amount of the UV-absorber ranges from about 0.01 % to 1% by weight, calculated to the total composition. Attention should be paid to the stability and solubility especially when using UV filter as salts, e.g. anionic UV filter salts.

The composition may comprises pyrrolidone carboxylic acid and/or its salts. In principal any pyrrolidone carboxylic acid salt is suitable within the meaning of the present invention. Suitable non-limiting examples are aluminium, calcium, copper, magnesium, potassium, sodium and zinc salts of pyrrolidone carboxylic acid. Preferred are aluminium, calcium, magnesium, potassium and sodium salts and more preferred are sodium and potassium salts. The most preferred is sodium salt.

The total concentration of pyrrolidone carboxylic acid and/or its salts is in the range of 0.01 to 10%, preferably 0.05 to 7.5%, more preferably 0.1 to 5% and most preferably 0.2 to 3% by weight calculated to the total composition.

The compositions may comprise further compounds which are customarily found in such compositions such as fragrance, neutralizing agents, solubilizing agents, preservatives, etc.

The following examples are to illustrate the invention but not to limit it.

### Example 1

The following compositions were prepared.

| | % by weight | | | | |
|---|---|---|---|---|---|
| | A | B | C | D | E |
| Acrylates/Octylacrylamide Copolymer | 20 | 20 | - | 20 | - |
| Aminomethylpropanol | 5 | 5 | - | 5 | - |
| Octyldodecyl PCA | 15 | - | 15 | - | - |
| Dimethicone | - | - | - | 15 | 15 |
| Ethanol | q.s. to 100 | | | | |

| | % by weight | | | | |
|---|---|---|---|---|---|
| | A | B | C | D | E |
| Acrylates/Octylacrylamide Copolymer | 20 | 20 | - | 20 | - |
| Octyldodecyl PCA | 15 | - | 15 | - | - |
| Dimethicone | - | - | - | 15 | 15 |
| Ethanol | q.s. to 100 | | | | |

The composition A is according to the present invention and the other compositions B to E are comparative examples.

The compositions were filled into an aerosol can as follows:

| | % by weight |
|---|---|
| The solution of Example 1 | 20.0 |
| n-pentane | 20.0 |
| Hydrofluorocarbon 152a | 60.0 |

For this purpose, the hair streak weighing approximately 2 g was washed first with a commercially available cleansing composition and dried with a hair dryer. The dry hair was applied the aerosol compositions above onto hair at a composition to hair weight ratio of 1:2 The hair steaks were left to dry in the air and the bending force and the shine were measured as disclosed below.

The bending force of the hair was measured with a zwick machine and expressed as the force (in milliNewton - mN). The machine measured the force needed to push down the streak between two rods. The machine is commercially available from Zwick/Roell Company and includes a manual. It was used according to its manual.

The shine measurement was carried out with the Samba Hair System from Bosa Nova Technologies via polarized illumination. The hair tress was illuminated and the specular reflection was measured. This gives information about the gloss of the hair.

The shine factor was calculated with the Reich Robins method.

The following results were obtained

| Composition | Bending force (mN) | Shine Factor |
|---|---|---|
| A | 0.62 | 30.45 |
| B | 0.48 | 11.32 |
| C | 0.14 | 17.50 |
| D | 0.29 | 22.49 |
| E | 0.09 | 15.60 |

It should be noted, for the bending force, the higher the number, the higher the hold of the style. For the shine factor, in the same way, the higher the number, the higher the shine.

From the above results, it is beyond any doubt that the composition according to the present invention has the highest score in bending force, indicating clearly the best hold and the highest score in shine factor indicating the best shine. It must be pointed out that silicones are known to enhance shine of hair; however, they even failed to enhance shine as good as the inventive composition. Furthermore, the bending force of the polymer film is reduced dramatically in the presence of dimethicone, however, presence of PCA ester increased the bending force showing clearly the better hold.

The results obtained with the individual compounds do not provide any indication to predict the combined effect.

### Example 2

| | % by weight |
|---|---|
| Acrylates/Octylacrylamide Copolymer | 4.0 |
| Aminomethylpropanol | 1.0 |
| Octyldodecyl PCA | 3.0 |
| Ethanol | q.s. to 100 |

The above composition was applied in the same way as in Example 1 using a mechanical spraying device (pump spray) and the bending force and shine factor measurements showed and confirmed the same effects.

The following examples are within the scope of the present invention and showed the similar effects on the hair.

### Example 3

| | % by weight |
|---|---|
| Acrylates/Octylacrylamide Copolymer | 20.0 |
| Aminomethylpropanol | 5.0 |
| Octyldodecyl PCA | 15.0 |
| Ethanol | q.s. to 100 |

The composition was filled into an aerosol can as follows:

| | % by weight |
|---|---|
| The solution of Example 3 | 20.0 |
| n-pentane | 20.0 |
| Dimethylether (DME) | 60.0 |

### Example 4

| | % by weight |
|---|---|
| VP/ Vinylcaprolactam/DMAPA Acrylates Copolymer | 20.0 |
| Octyldodecyl PCA | 15.0 |
| Ethanol | q.s. to 100 |

The composition was filled into an aerosol can as follows:

| | % by weight |
|---|---|
| The solution of Example 4 | 20.0 |
| n-pentane | 20.0 |
| Hydrofluorocarbon 152a | 60.0 |

The same composition was also filled into a second aerosol can as follows:

| | % by weight |
|---|---|
| The solution of Example 4 | 30.0 |
| Propane/butane | 40.0 |
| DME | 30.0 |

### Example 5

| | % by weight |
|---|---|
| VP/ VA Copolymer | 20.0 |
| Octyldodecyl PCA | 15.0 |
| Ethanol | q.s. to 100 |

The composition was filled into an aerosol can as follows:

| | % by weight |
|---|---|
| The solution of Example 5 | 20.0 |
| n-pentane | 20.0 |
| Hydrofluorocarbon 152a | 60.0 |

The same composition was also filled into a second aerosol can as follows:

| | % by weight |
|---|---|
| The solution of Example 5 | 30.0 |
| Propane/butane | 40.0 |
| DME | 30.0 |

### Example 6

| | % by weight |
|---|---|
| Acrylates/Octylacrylamide Copolymer | 20.0 |
| Aminomethylpropanol | 5.0 |
| Octyldodecyl PCA | 15.0 |
| Propyleneglycol | 1.0 |
| Ethanol | q.s. to 100 |

### Example 7

| | % by weight |
|---|---|
| Acrylates/Octylacrylamide Copolymer | 20.0 |
| Aminomethylpropanol | 5.0 |
| Octyldodecyl PCA | 15.0 |
| Benzopheneone 3 | 1.0 |
| Ethanol | q.s. to 100 |

### Example 8

| | % by weight |
|---|---|
| Acrylates/Octylacrylamide Copolymer | 20.0 |
| Aminomethylpropanol | 5.0 |
| Octyldodecyl PCA | 15.0 |
| Dimethicone | 1.0 |
| Ethanol | q.s. to 100 |

### Example 9

| | % by weight |
|---|---|
| Acrylates/Octylacrylamide Copolymer | 20.0 |
| Aminomethylpropanol | 5.0 |
| Octyldodecyl PCA | 15.0 |
| Wheat germ oil | 1.0 |
| Ethanol | q.s. to 100 |

### Example 10

| | % by weight |
|---|---|
| Acrylates/Octylacrylamide Copolymer | 20.0 |
| Aminomethylpropanol | 5.0 |
| Octyldodecyl PCA | 15.0 |
| Isopropyl myristate | 1.0 |
| Ethanol | q.s. to 100 |

### Example 11

| | % by weight |
|---|---|
| Acrylates/Octylacrylamide Copolymer | 20.0 |
| Aminomethylpropanol | 5.0 |
| Octyldodecyl PCA | 15.0 |
| Phenyl trimethicone | 1.0 |
| Ethanol | q.s. to 100 |

### Example 12

| | % by weight |
|---|---|
| VP/VA Copolymer | 20.0 |
| Octyldodecyl PCA | 15.0 |
| Propyleneglycol | 1.0 |
| Ethanol | q.s. to 100 |

### Example 13

| | % by weight |
|---|---|
| VP/VA Copolymer | 20.0 |
| Octyldodecyl PCA | 15.0 |
| Benzopheneone 3 | 1.0 |
| Ethanol | q.s. to 100 |

### Example 14

| | % by weight |
|---|---|
| VP/VA Copolymer | 20.0 |
| Octyldodecyl PCA | 15.0 |
| Dimethicone | 1.0 |
| Ethanol | q.s. to 100 |

### Example 16

| | % by weight |
|---|---|
| VP/VA Copolymer | 20.0 |
| Octyldodecyl PCA | 15.0 |
| Wheat germ oil | 1.0 |
| Ethanol | q.s. to 100 |

### Example 17

| | % by weight |
|---|---|
| VP/VA Copolymer | 20.0 |
| Octyldodecyl PCA | 15.0 |
| Isopropyl myristate | 1.0 |
| Ethanol | q.s. to 100 |

### Example 18

| | % by weight |
|---|---|
| VP/VA Copolymer | 20.0 |
| Octyldodecyl PCA | 15.0 |
| Phenyl trimethicone | 1.0 |
| Ethanol | q.s. to 100 |

### Example 19

| | % by weight |
|---|---|
| VP/VA Copolymer | 10.0 |
| Acrylates/Octylacrylamide Copolymer | 10.0 |
| Aminomethylpropanol VP/ Vinylcaprolactam/DMAPA | 2.5 |
| Acrylates Copolymer | 10.0 |
| Octyldodecyl PCA | 15.0 |
| Isopropyl myristate | 0.1 |
| Phenyl trimethicone | 0.1 |
| Propylene glycol | 0.1 |
| Benzophenone-3 | 0.5 |
| Alcoholic Chamomile extract | 1.0 |
| Ethanol | q.s. to 100 |

The Examples 6 to 19 were used as follows:

### Non-aerosol compositions:

| | % by weight |
|---|---|
| The solution of Examples | 20 |
| Ethanol | 80 |

The non-aerosol compositions were applied onto hair using a mechanical spraying device (pump spray).

### Aerosol compositions

### Option 1:

| | % by weight |
|---|---|
| The solution of Examples | 20.0 |
| n-pentane | 20.0 |
| Hydrofluorocarbon 152a | 60.0 |

### Option 2:

| | % by weight |
|---|---|
| The solution of Examples | 30.0 |
| Hydrofluorocarbon 152a | 70.0 |

### Option 3:

| | % by weight |
|---|---|
| The solution of Examples | 20.0 |
| n-pentane | 20.0 |
| DME | 60.0 |

### Option 4:

| | % by weight |
|---|---|
| The solution of Example | 30.0 |
| Propane/butane | 40.0 |
| DME | 30.0 |

## Claims

1. A composition for keratin fibers, especially for human hair, **characterized in that** it comprises, in a cosmetically acceptable medium, one or more hair setting film forming polymers and one or more pyrrolidone carboxylic acid ester according to general structure 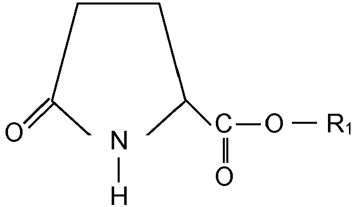
wherein R₁ is a straight or branched, saturated or unsaturated alkyl chain with 1 to 22 C atoms, phenyl, phenyl alkyl with alkyl chain length of 1 to 4 C atoms, phenyl alkoxy group with alkyl chain length of 1 to 4 C atoms and phenoxy alkyl group with alkyl chain length of 1 to 4 C atoms,
wherein the composition comprises water less than 5% by weight, calculated to the total composition,
wherein the pyrrolidone carboxylic ester is octyldodecyl pyrrolidone carboxylic acid

2. The composition according to claim 1 **characterized in that** hair setting film forming polymers is selected from anionic, non-ionic, cationic and amphoteric ones and their mixtures.

3. The composition according to any of the preceding claims **characterized in that** it comprises one or more hair setting film forming polymers at a total concentration in the range of 0.1 to 25% by weight, preferably 0.5 to 20% by weight, more preferably 1.0 to 15% by weight and most preferably 2 to 15% by weight, in particular 3 to 15% by weight, calculated to the total of the composition.

4. The composition according to any of the preceding claims **characterized in that** it comprises one or more pyrrolidone carboxylic acid ester at a total concentration in the range of 0.01 to 15%, preferably 0.1 to 10%, more preferably 0.25 to 7.5% and most preferably 0.5 to 5% by weight, calculated to the total composition.

5. The composition according to any of the preceding claims **characterized in that** it comprises one or more hair setting film forming anionic polymers preferably comprised as partially or completely (100%) neutralized with an alkalizing agent selected from alkanolamines, preferably it is aminomethylpropanol.

6. The composition according to any of the preceding claims **characterized in that** it comprises one or more organic solvents, preferably selected from ethanol, isopropanol and propanol, more preferably it is ethanol.

7. The composition according to claim 6 **characterized in that** it comprises one or more organic solvents at a concentration in the range of 30 to 95%, preferably 40 to 90%, more preferably 45 to 85%, most preferably 50 to 80% and in particular 60 to 75% by weight calculated to the total composition.

8. The composition according to any of the preceding claims **characterized in that** it comprises one or more propellants, preferably at a concentration in the range of 10 to 80%, more preferably 20 to 70%, even more preferably 30 to 65% and most preferably 40 to 60% by weight, calculated to the total composition.

9. The composition according to any of the preceding claims **characterized in that** one or more propellants is (are) selected from n-butane, i-butane, propane, butane dimethylether (DME), 1,1-difluoro ethane (Hydrofluorocarbon 152a) and tetrafluoroethane.

10. The composition according to any of the preceding claims **characterized in that** it comprises water less than 3% by weight, more preferably less than 1 % by weight and most preferably less than 0.5% by weight, calculated to the total composition, in particular it is anhydrous.

11. The composition according to any of the preceding claims **characterized in that** it comprises one or more of the following ingredients
- One or more polyols
- One or more UV filter
- One or more oil selected from natural and synthetic oils, and
- One or more natural plant extracts.

12. The use of the composition according to any of the preceding claims for providing keratin fibers, especially human hair, setting, hold and shine.

13. The method of setting and shine enhancing and providing hold to keratin fibers, especially human hair wherein the composition according to claim 1 to 11 is applied to dry hair, preferably by spraying.

14. A kit for keratin fibers, especially human hair comprising the composition according to claims 1 to 11.

## Patentansprüche

1. Zusammensetzung für Keratinfasern, insbesondere für menschliches Haar, **dadurch gekennzeichnet, dass** sie in einem kosmetisch verträglichen Träger ein oder mehrere haarfestigende, filmbildende Polymere und einen oder mehrere Pyrrolidoncarbonsäureester der allgemeinen Struktur enthält 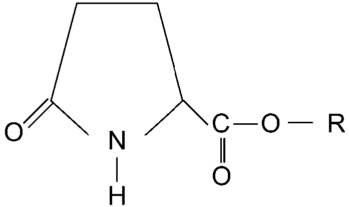
wobei R1 eine geradkettige oder verzweigte, gesättigte oder ungesättigte Alkylkette mit 1 bis 22 Kohlenstoffatomen, eine Phenylgruppe, eine Phenylalkylgruppe mit einer Alkylkette von 1 bis 4 Kohlenstoffatomen, eine Phenylalkoxygruppe mit einer Alkylkette von 1 bis 4 Kohlenstoffatomen oder eine Phenoxyalkylgruppe mit einer Alkylkette von 1 bis 4 Kohlenstoffatomen ist,
wobei die Zusammensetzung weniger als 5 Gew.-% Wasser, bezogen auf die Gesamtzusammensetzung, enthält,wobei es sich bei dem Pyrrolidoncarbonsäureester um Octyldodecylpyrrolidoncarbonsäure handelt.

2. Die Zusammensetzung nach Anspruch 1, **dadurch gekennzeichnet, dass** die haarfestigenden, filmbildenden Polymere aus anionischen, nichtionischen, kationischen und amphoteren Polymeren sowie deren Mischungen ausgewählt sind.

3. Die Zusammensetzung nach einem der vorstehenden Ansprüche, **dadurch gekennzeichnet, dass** sie ein oder mehrere haarfestigende, filmbildende Polymere in einer Gesamtkonzentration im Bereich von 0.1 bis 25 Gew.-%, vorzugsweise 0.5 bis 20 Gew.-%, stärker bevorzugt 1.0 bis 15 Gew.-% und am stärksten bevorzugt 2 bis 15 Gew.-%, insbesondere 3 bis 15 Gew.-%, bezogen auf die Gesamtmenge der Zusammensetzung.

4. Die Zusammensetzung gemäß einem der vorstehenden Ansprüche, **dadurch gekennzeichnet, dass** sie einen oder mehrere Pyrrolidoncarbonsäureester in einer Gesamtkonzentration im Bereich von 0.01 bis 15 Gew.-%, vorzugsweise 0.1 bis 10 Gew.-%, noch bevorzugter 0.25 bis 7.5 Gew.-% und am meisten bevorzugt 0.5 bis 5 Gew.-%, bezogen auf die Gesamtzusammensetzung, enthält.

5. Die Zusammensetzung gemäß einem der vorstehenden Ansprüche, **dadurch gekennzeichnet, dass** sie ein oder mehrere filmbildende, anionische Haarfestigungs-Polymere enthält, die vorzugsweise teilweise oder vollständig (100 %) mit einem Alkalisierungsmittel neutralisiert sind, das aus Alkanolaminen ausgewählt ist, vorzugsweise Aminomethylpropanol.

6. Die Zusammensetzung gemäß einem der vorstehenden Ansprüche, **dadurch gekennzeichnet, dass** sie ein oder mehrere organische Lösungsmittel enthält, die vorzugsweise aus Ethanol, Isopropanol und Propanol ausgewählt sind, noch bevorzugter ist es Ethanol.

7. Die Zusammensetzung nach Anspruch 6, **dadurch gekennzeichnet, dass** sie ein oder mehrere organische Lösungsmittel in einer Konzentration im Bereich von 30 bis 95 Gew.-%, vorzugsweise 40 bis 90 Gew.-%, noch bevorzugter 45 bis 85 Gew.-%, am meisten bevorzugt 50 bis 80 Gew.-% und insbesondere 60 bis 75 Gew.-%, bezogen auf die Gesamtzusammensetzung, enthält.

8. Die Zusammensetzung gemäß einem der vorstehenden Ansprüche, **dadurch gekennzeichnet, dass** sie ein oder mehrere Treibmittel enthält, vorzugsweise in einer Konzentration im Bereich von 10 bis 80 Gew.-%, noch bevorzugter von 20 bis 70 Gew.-%, am meisten bevorzugt von 30 bis 65 Gew.-% und am allermeisten bevorzugt von 40 bis 60 Gew.-%, bezogen auf die Gesamtzusammensetzung.

9. Die Zusammensetzung gemäß einem der vorstehenden Ansprüche, **dadurch gekennzeichnet, dass** ein oder mehrere Treibmittel aus n-Butan, i-Butan, Propan, Butandimethylether (DME), 1,1-Difluorethan (Fluorkohlenwasserstoff 152a) und Tetrafluorethan ausgewählt ist (sind).

10. Die Zusammensetzung gemäß einem der vorstehenden Ansprüche, **dadurch gekennzeichnet, dass** sie weniger als 3 Gew.-%, vorzugsweise weniger als 1 Gew.-% und am bevorzugtesten weniger als 0.5 Gew.-% Wasser, bezogen auf die Gesamtzusammensetzung, enthält; insbesondere ist sie wasserfrei.

11. Die Zusammensetzung gemäß einem der vorstehenden Ansprüche, **dadurch gekennzeichnet, dass** sie einen oder mehrere der folgenden Bestandteile enthält
- Ein oder mehrere Polyole
- Ein oder mehrere UV-Filter
- ein oder mehrere Öle, ausgewählt aus natürlichen und synthetischen Ölen, und
- Ein oder mehrere natürliche Pflanzenextrakte.

12. Verwendung der Zusammensetzung gemäß einem der vorstehenden Ansprüche zur Verleihung von Festigkeit, Halt und Glanz an Keratinfasern, insbesondere an menschlichem Haar.

13. Verfahren zur Formgebung, Glanzverstärkung und Fixierung von Keratinfasern, insbesondere von menschlichem Haar, bei dem die Zusammensetzung gemäß Anspruch 1 bis 11 auf das trockene Haar aufgetragen wird, vorzugsweise durch Aufsprühen.

14. Ein Kit für Keratinfasern, insbesondere für menschliches Haar, das die Zusammensetzung gemäß den Ansprüchen 1 bis 11 umfasst.

## Revendications

1. Composition destinée aux fibres kératiniques, en particulier aux cheveux humains, **caractérisée en ce qu'**elle comprend, dans un milieu cosmétiquement acceptable, un ou plusieurs polymères filmogènes fixateurs de coiffure et un ou plusieurs esters d'acide pyrrolidone-carboxylique répondant à la structure générale 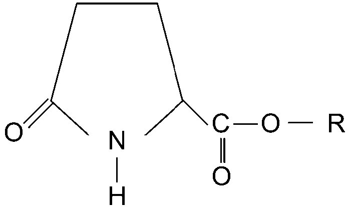
dans laquelle R1 représente une chaîne alkyle linéaire ou ramifiée, saturée ou insaturée, comportant de 1 à 22 atomes de carbone, un groupe phényle, un groupe phénylalkyle dont la chaîne alkyle comporte de 1 à 4 atomes de carbone, un groupe phénylalkoxy dont la chaîne alkyle comporte de 1 à 4 atomes de carbone et un groupe phénoxyalkyle dont la chaîne alkyle comporte de 1 à 4 atomes de carbone,
dans laquelle la composition contient moins de 5 % en poids d'eau, par rapport à la composition totale, et dans laquelle l'ester carboxylique de pyrrolidone est l'acide octyldodécylpyrrolidone-carboxylique.

2. Composition selon la revendication 1, **caractérisée en ce que** les polymères filmogènes fixateurs de coiffure sont choisis parmi les polymères anioniques, non ioniques, cationiques et amphotères, ainsi que leurs mélanges.

3. Composition selon l'une quelconque des revendications précédentes, **caractérisée en ce qu'**elle comprend un ou plusieurs polymères filmogènes fixateurs de coiffure à une concentration totale comprise entre 0.1 et 25 % en poids, de préférence entre 0.5 et 20 % en poids, de préférence de 1.0 à 15 % en poids et, de manière encore plus préférée, de 2 à 15 % en poids, en particulier de 3 à 15 % en poids, par rapport au poids total de la composition.

4. Composition selon l'une quelconque des revendications précédentes, **caractérisée en ce qu'**elle comprend un ou plusieurs esters d'acide pyrrolidone-carboxylique à une concentration totale comprise entre 0.01 et 15 %, de préférence entre 0.1 et 10 %, plus préférablement entre 0.25 et 7.5 % et, de manière encore plus préférable, entre 0.5 et 5 % en poids, calculée par rapport à la composition totale.

5. Composition selon l'une quelconque des revendications précédentes, **caractérisée en ce qu'**elle comprend un ou plusieurs polymères anioniques filmogènes pour la mise en forme des cheveux, de préférence partiellement ou totalement (100 %) neutralisés par un agent alcalinisant choisi parmi les alcanolamines, de préférence l'aminométhylpropanol.

6. Composition selon l'une quelconque des revendications précédentes, **caractérisée en ce qu'**elle comprend un ou plusieurs solvants organiques, choisis de préférence parmi l'éthanol, l'isopropanol et le propanol, et de préférence encore l'éthanol.

7. Composition selon la revendication 6, **caractérisée en ce qu'**elle comprend un ou plusieurs solvants organiques à une concentration comprise entre 30 et 95 %, de préférence entre 40 et 90 %, plus préférablement entre 45 et 85 %, encore plus préférablement entre 50 et 80 % et en particulier entre 60 et 75 % en poids, par rapport à la composition totale.

8. Composition selon l'une quelconque des revendications précédentes, **caractérisée en ce qu'**elle comprend un ou plusieurs agents propulseurs, de préférence à une concentration comprise entre 10 et 80 %, de préférence entre 20 et 70 %, de préférence encore entre 30 et 65 % et de préférence entre 40 et 60 % en poids, par rapport à la composition totale.

9. Composition selon l'une quelconque des revendications précédentes, **caractérisée en ce qu'**un ou plusieurs agents propulseurs sont choisis parmi le n-butane, l'isobutane, le propane, l'éther diméthylique de butane (DME), le 1,1-difluoroéthane (hydrofluorocarbone 152a) et le tétrafluoroéthane.

10. Composition selon l'une quelconque des revendications précédentes, **caractérisée en ce qu'**elle contient moins de 3 % en poids d'eau, de préférence moins de 1 % en poids et, de manière encore plus préférable, moins de 0.5 % en poids, par rapport au poids total de la composition ; elle est notamment anhydre.

11. Composition selon l'une quelconque des revendications précédentes, **caractérisée en ce qu'**elle comprend un ou plusieurs des ingrédients suivants
- Un ou plusieurs polyols
- Un ou plusieurs filtres UV
- une ou plusieurs huiles choisies parmi les huiles naturelles et synthétiques, et
- Un ou plusieurs extraits végétaux naturels.

12. Utilisation de la composition selon l'une quelconque des revendications précédentes pour conférer aux fibres kératiniques, en particulier aux cheveux humains, une tenue, une fixation et de la brillance.

13. Procédé permettant de fixer, de rehausser la brillance et d'assurer la tenue des fibres kératiniques, en particulier des cheveux humains, dans lequel la composition selon les revendications 1 à 11 est appliquée sur des cheveux secs, de préférence par pulvérisation.

14. Kit destiné aux fibres de kératine, en particulier aux cheveux humains, comprenant la composition selon les revendications 1 à 11.
